# FASCICULE DE BREVET EUROPEEN

(11) **EP 1 185 613 B1**
(45) Date de publication et mention de la délivrance du brevet: **06.10.2004**
(21) Numéro de dépôt: 00929611.2
(22) Date de dépôt: 12.05.2000
(51) Int. Cl.: C12M 1/107

(54) **PROCEDE DE TRAITEMENT DES MELANGES BIODIGESTIBLES DE SOLIDES ET LIQUIDES**
VERFAHREN ZUR BEHANDLUNG VON BIOLOGISCH ABBAUBARE STOFF/FLÜSSIGKEIT MISCHUNGEN
METHOD FOR TREATING BIODIGESTIBLE MIXTURES OF SOLIDS AND LIQUIDS

(30) Priorité: 17.05.1999 FR 9906220
(43) Date de publication de la demande: 13.03.2002
(73) Titulaire: Rassak, Denis, F-78430 Louveciennes (FR)
(72) Inventeur: Rassak, Denis, F-78430 Louveciennes (FR)
(86) Numéro de dépôt international: PCT/FR2000/001292
(87) Numéro de publication internationale: WO 2000/070010

(56) Documents cités:
- EP-A- 0 038 759
- EP-A- 0 291 425
- FR-A- 2 518 116
- GB-A- 2 230 004

## Description

La présente invention concerne un procédé de traitement des mélanges biodigestibles de solides et liquides. Elle est relative à des perfectionnements apportés aux procédés et installations de traitement des matières biodigestibles de toute nature mettant en oeuvre la Digestion Anaérobie Méthanogène autrement appelée Méthanisation.Les matières traitées sont des mélanges de matières organiques digestibles, d'eau, et de bien d'autres matériaux, matières organiques non digestibles, minéraux, métaux, détritus divers. De nombreux procédés de traitement ont été développés, qui associent des processus physiques, chimiques et biologiques mis en oeuvre par des equipements spécialisés.

Ils visent généralement à :
Stabiliser - désodoriser les matières pour en faciliter le réemploi et/ou la mise en dépôt et/ou le rejet en milieu naturel ;
Récupérer le potentiel énergétique ;
Récupérer le potentiel recyclable en Agriculture ;
Isoler les matériaux réutilisables ;
Minimiser la production des déchets ultimes ;

Stabilisation - désodorisation sont obtenus par des processus biologiques parmi lesquels la méthanisation permet de récupérer le potentiel énergétique de la partie biodégradable. Des processus physiques de séparation solides - liquides, parfois complétés d'épisodes chimiques, et des opérations de tri densimétrique et granulométrique sont utilisés pour isoler la part recyclable en agriculture, les matériaux réutilisables, tels que métaux et matières plastiques, entre eux et d'avec des déchets et effluents à éliminer.

La méthanisation correspond à la digestion anaérobie en milieu aqueux de la partie biodigestible des matières traitées ; des familles bactériennes attaquent les solides, les solubilisent en partie ; d'autres familles fixées sur les solides, digèrent les matières biodigestibles en solution ; le résultat macroscopique est la disparition de matière sous forme de biogaz, mélange de méthane, dioxyde de carbone et diverses impuretés malodorantes qui seront détruites par la combustion du biogaz.

Une charge admise à la méthanisation doit contenir assez d'eau pour assurer l'anaérobiose et obtenir PH et concentrations des matières en solution convenables ; après traitement, cette charge est proportionnellement plus humide, et doit faire l'objet d'une séparation solides - liquides avant que les parts recyclables, réutilisables et destinées à l'élimination puissent être isolées les unes des autres.

La demande de certificat d'addition au brevet E.P. -A - 0038759, publiée sous numéro FR-A-2518116 décrit un procédé dans lequel une charge constituée d'un mélange de solides et liquides est traitée dans une installation de méthanisation qui vise à séparer les solides et liquides simultanément à la digestion ; une première enceinte reçoit la charge ; elle est munie d'un système de drainage des liquides qui les collecte vers une seconde enceinte, d'où ils sont extraits soit pour être recyclés vers la première enceinte, en particulier pour noyer les matières entrantes, soit pour être évacués hors de l'installation ; un résidu consolidé peu aqueux est extrait de la première enceinte. La demande FR-A-2518116 présente quelques perfectionnements au dessin des installations décrites par le Brevet principal E.P. - A -0038759.

L'expérience a montré que ce principe fonctionne pourvu que d'autres perfectionnements soient apportés au dessin des installations, que soit améliorée la manière dont les matières à traiter sont préparées et introduites dans les enceintes de méthanisation, enfin que soit améliorée la conduite du transit de la charge dans l'installation. Ce principe permet alors de développer un procédé de traitement de la plus large variété de mélanges biodigestibles de solides et liquides, permettant de remplir les objectifs du traitement rappelés ci-avant plus complètement et pour moins cher qu'avec les procédés connus utilisables à cet effet.

La présente invention a pour objet un procédé de traitement de mélanges biodigestibles de solides et liquides qui apporte les perfectionnements au dessin des installations et à la manière dont elles sont utilisées pour traiter les matières introduites par rapport au principe et aux dessins d'installation décrits par la demande FR-A-2518116 et le Brevet principal E.P.-A-0038759.

Grâce à la séparation liquides - solides concomitante à la digestion, on peut prélever dans l'installation de méthanisation des liquides en toute quantité pour préparer la charge admise à la méthanisation, quelle que soit l'humidité des matières brutes entrantes.

Les matières après méthanisation sont transformées en 3 sortants :
des Solides Résiduels rassemblés en un compost anaérobie essoré, désigné ci-après par résidu très peu aqueux
des Liquides Résiduels très peu chargés de matières en suspension du Biogaz

Lorsque les matières traitées sont peu humides, il peut être nécessaire d'ajouter de l'eau de procédé et il n'y a pas de liquide résiduel ; dans le cas de matières très humides, comme un effluent plus ou moins boueux, la méthanisation agit à la fois comme traitement biologique et moyen de séparer solides et eau interstitielle.

La préparation de la charge vise à obtenir un mélange de solides et liquides, au besoin avec addition d'eau et/ou de solides «de procédé », remplissant les conditions suivantes :
PRESENCE DE SOLIDES à la granulométrie fine, grossière, et d'assez grande taille, biodigestibles ou non, en dimensions et proportions bien contrôlées ; les solides de taille significative doivent être en quantité suffisante pour obtenir une charge admise à la méthanisation perméable pendant une partie de son transit et essorable lors de son extraction.
LA CHARGE ADMISE A LA METHANISATION doit être biodigestible à l'entrée et pendant son transit. Outre un contenu en constituants digestibles suffisant, elle doit présenter un PH convenable, ainsi que des concentrations en éléments potentiellement inhibiteurs de la digestion suffisamment basses.
LA PREPARATION DE LA CHARGE peut prendre tout ou partie des formes suivantes pour tout ou partie des matières traitées :
   PREPARATION MECANIQUE, par broyage, criblage, élimination d'éléments indésirables, mélange des matières entre elles et avec des additions de procédé.
   PREPARATION HYDRAULIQUE des solides et substrats concentrés, par mélange avec des liquides prélevés à la méthanisation pour séparer les matières brutes ou mécaniquement préparées en 3 parties :
      les matières de faible densité, qui flottent
      les matières de haute densité, qui décantent
      les matières en solution ou suspension
   On observera que les matières organiques biodigestibles sont généralement solubles ou contenues par des tissus animaux et végétaux de densité voisine de 1, alors que les légers et les denses ne sont généralement pas digestibles.
   PREPARATION BIOCHIMIQUE des matières brutes ou préparées mécaniquement ou hydrauliquement, par mélange avec des liquides prélevés à la méthanisation. Les effets attendus sont :
      MELANGE INTIME des constituants de la charge entre eux et avec les liquides prélevés à la méthanisation pour éliminer l'air de la charge, et la rendre pompable par une pompe à liquides chargés.
      ENSEMENCEMENT de la charge en ferments adaptés actifs
      CORRIGER LE PH ET/OU LA CONCENTRATION en éléments inhibants des matières entrantes grâce au potentiel biochimique des liquides recyclés.

Après méthanisation, le résidu très peu aqueux peut être introduit directement dans une étuve chauffée avec tout ou partie du potentiel calorifique du biogaz, pour l'hygiéniser (neutraliser les germes pathogènes et semences parasites) et le sécher, au moins partiellement.

Le résidu hygiénisé séché peut être affiné par tout moyen de sélection densimétrique et granulométrique afin d'isoler le compost anaérobie affiné, recyclable en milieu naturel, voire en agriculture s'il n'est pas chargé de trop de métaux lourds, les matériaux réutilisables tels que métaux et plastiques, et les déchets à éliminer.

L'ensemble d'affinage peut être utilisé également pour traiter les légers et denses arrêtés à la préparation hydraulique.

L'affinage permet de séparer les solides de taille significative du résidu très peu aqueux pour les recycler comme addition solide de procédé à une matière entrante, par exemple un effluent boueux qui ne contient que des solides pulvérulents.

Les liquides résiduels, très peu chargés de matières en suspension, peuvent être envoyés directement à une station d'épuration d'eaux usées associée à la méthanisation ; cette station peut traiter les liquides résiduels seuls, ou en mélange avec d'autres effluents, les boues produites étant envoyées directement à la méthanisation. Par cette association de la méthanisation et de la station d'épuration, le procédé de traitement objet de l'invention, assure l'élimination complète et des liquides résiduels de la méthanisation et des boues de la station d'épuration transformés respectivement en eau épurée rejetable en milieu naturel et résidu très peu aqueux de méthanisation.

L'INSTALLATION DE METHANISATION conforme à l'invention se compose de :
UNE CUVE D'HOMOGENEÏSATION de la charge et d'ALIMENTATION qui reçoit la totalité des solides et substrats concentrés, tout ou partie des substrats dilués et des liquides prélevés dans l'installation.
UNE ENCEINTE A SOLIDES ET SUBSTRATS CONCENTRES qui reçoit la totalité de la charge ; la charge comprend les matières provenant de la cuve d'homogénéïsation - alimentation, des substrats dilués ne transitant pas par cette cuve et des liquides prélevés dans l'installation et recyclés directement à l'enceinte.
   La charge est introduite par un unique point situé en haut de l'enceinte, en continu ou discontinu. Elle transite verticalement, par gravité.
   L'enceinte est munie sur les flancs et le fond d'un système de drainage des liquides circulants , pouvant permettre une collecte sélective des liquides selon la position où ils ont été drainés ; elle est munie en partie basse d'un système de récolte des solides et substrats concentrés résiduels qui essore et extrait de l'enceinte un résidu très peu aqueux en maintenant l'étanchéïté aux liquides et aux gaz entre l'intérieur de l'enceinte et l'atmosphère extérieure ; cette extraction peut être continue ou discontinue.
   Le transit de la charge dans l'enceinte est piloté par les entrées et sorties de façon à ce que deux zones se constituent sur la hauteur de l'enceinte :
   LA ZONE SUPERIEURE est le siège d'une séparation solides/liquides rapide par flottation, décantation, et filtration par le lit de solides en place.
   LA ZONE INFERIEURE est le siège d'une séparation lente par filtration et libération de liquides circulants par digestion de matière biodigestible dans la masse en place.
UNE ENCEINTE A LIQUIDES, qui reçoit les liquides drainés depuis l'enceinte à solides et substrats concentrés par un dispositif de collecte pouvant acheminer séparément les liquides collectés à différents niveaux de l'enceinte à solides et substrats concentrés ; les mouvements de matières dans l'enceinte sont gravitaires et induits par les entrées et sorties ; par sélection des entrées selon l'origine des liquides et par des cloisonnements, des liquides résiduels très peu chargés de matières en suspension sont isolés de liquides destinés à être recyclés ; les liquides résiduels, lorsqu'il y en a, sont évacués par trop plein avec siphon d'isolement de l'intérieur de l'enceinte d'avec l'atmosphère extérieure.

Les enceintes des deux types sont chauffées de façon autonome pour porter et maintenir leur contenu à une température réglable dans le champ des températures de digestion dites mésophiles ou thermophiles.

L'installation de méthanisation conforme à l'invention peut comporter plus d'une Enceinte à Solides et Substrats Concentrés ; ces enceintes assurent la même fonction en parallèle ; il y a autant de points d'extraction de résidu très peu aqueux.

L'installation de méthanisation conforme à l'invention peut comporter plus d'une Enceinte à Liquides ; ces enceintes peuvent avoir deux à deux la même fonction ou des fonctions complémentaires ; le dispositif de collecte qui achemine les liquides drainés dans la (ou les) Enceintes (s) à Solides et Substrats Concentrés vers la (ou les) Enceintes à Liquides, et le système de liaison hydraulique entre les Enceintes à Liquides, lorsqu'il y en a plus d'une, sont agencés de façon à obtenir un parcours méthodique des liquides dans chaque Enceinte à Liquides, et entre les Enceintes à Liquides lorsqu'il y en a plusieurs. S'il y a rejet de liquides résiduels, il s'opère en un point unique de l'installation de méthanisation.

L'installation de méthanisation conforme à l'invention comporte un réseau de collecte du biogaz généré dans la (ou les) Enceinte(s) à Solides et Substrats concentré et dans la (ou les) Enceintes à Liquides réalisé de façon à ce que l'ensemble des volumes occupés par le biogaz, dans chacune des enceintes à Solides et Substrats concentré ou à Liquides et dans le réseau de collecte du biogaz, forme une phase gazeuse unique ; cette phase gazeuse est maintenue à pression constante par tout moyen connu approprié, gazomètre à cloche et joint hydraulique par exemple.

Selon le procédé, la préparation de la charge est réalisée de la façon suivante :
POUR UN MELANGE DE LIQUIDES ET SOLIDES DE TOUTES DIMENSIONS, les solides de grandes dimensions étant faiblement représentés, la préparation de charge est faite en totalité dans la cuve d'homogénéïsation - alimentation, sauf pour des substrats dilués et des liquides prélevés dans une Enceinte à Liquides qui peuvent être pompés dans la (ou les) Enceinte(s) à solides et substrats concentrés, éventuellement après mélange dans une unité de préparation biochimique.
POUR LES MELANGES CONTENANT BEAUCOUP DE SOLIDES DE GRANDES DIMENSIONS, une unité de préparation mécanique est prévue avant envoi soit à la préparation hydraulique, soit à la cuve d'homogénéïsation - alimentation.
POUR LES MELANGES CONTENANT BEAUCOUP DE LEGERS ET/OU DE DENSES, on dispose une unité de préparation hydraulique en amont de la cuve d'homogénéïsation - alimentation par mélange avec des liquides prélevés dans l'installation de méthanisation pour séparer les matières brutes ou mécaniquement préparées en 3 parties :
   les matières de faible densité, qui flottent
   les matières de haute densité, qui décantent
   les matières en solution ou suspension,
   qui seules sont envoyées à l'installation de méthanisation.
POUR LES SUBSTRATS LIQUIDES ET BOUES ne contenant que des solides pulvérulents, une addition de « solides de procédé», partiellement ou non biodigestibles, est faite au niveau de la cuve d'homogénéïsation alimentation,
QUANT A LA PREPARATION BIOCHIMIQUE, elle est effectuée dans l'un et/ou l'autre des dispositifs où des matières entrantes sont mélangées à des liquides issus de l'enceinte à liquides, cuve d'homogénéïsation alimentation, préparation hydraulique, préparation biochimique.

Il doit être bien entendu que les dispositifs décrits dans ce qui suit et représentés par les dessins annexés ne sont donnés que pour illustrer l'objet de l'invention et n'en constituent en aucune matière une limitation.

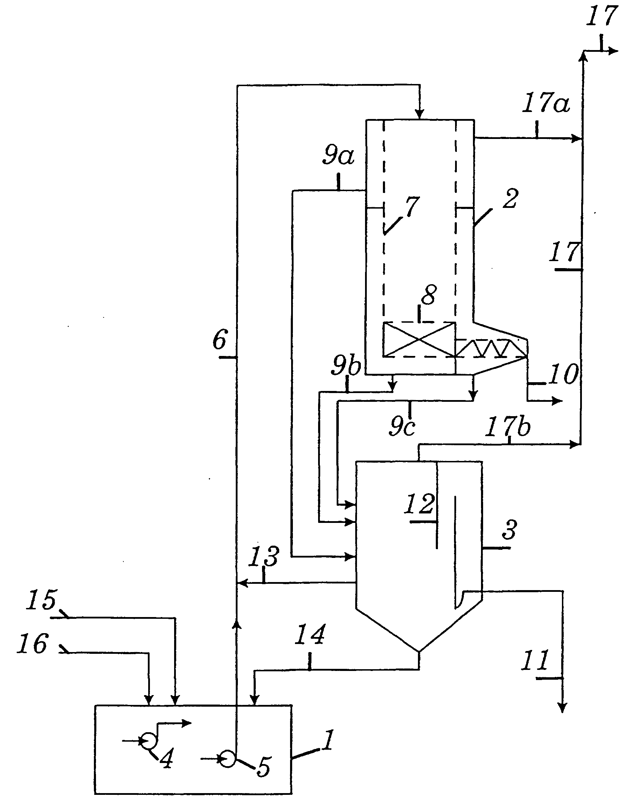

L'installation de méthanisation représentée sur la figure 1, conforme à la présente invention, comprend, non limitativement :
- une cuve (1) d'homogénéïsation alimentation équipée d'une pompe hacheuse d'homogénéïsation (4) et d'une pompe d'alimentation (5) qui reçoit :
   - des liquides issus de l'enceinte à liquide (3) par la ligne (14) ;
   - des solides et substrats concentrés par la ligne (16),
   - des substrats dilués par la ligne (15),
- une enceinte à solides et substrats concentrés (2) qui reçoit la charge issue de (1) par la ligne (6) et des liquides recyclés depuis l'enceinte à liquides (3) par les lignes (13) puis (6).
   l'enceinte (2) est équipée d'un système de drainage (7) sur les flancs et le fond, d'un dispositif (8) de récolte et extraction de résidu très peu aqueux déposé sur le transporteur (10).
   Les liquides drainés depuis (2) sont acheminés par le système de collecte (9) à l'enceinte à liquides (3) ; ce système (9) collecte séparément les liquides issus de la zone supérieure de (2) par (9a), les liquides issus de la zone inférieure de (2) par (9b), les liquides d'essorage du résidu très peu aqueux par (9c).
   L'enceinte à liquide (3) est munie d'un cloisonnement interne (12) assurant un cheminement méthodique des liquides, permettant d'isoler des liquides résiduels le moins chargés possible en matières en solution et suspension, pour être évacués hors de (3) par trop plein s'écoulant dans la ligne (11).
   Le biogaz est recueilli par le collecteur (17).

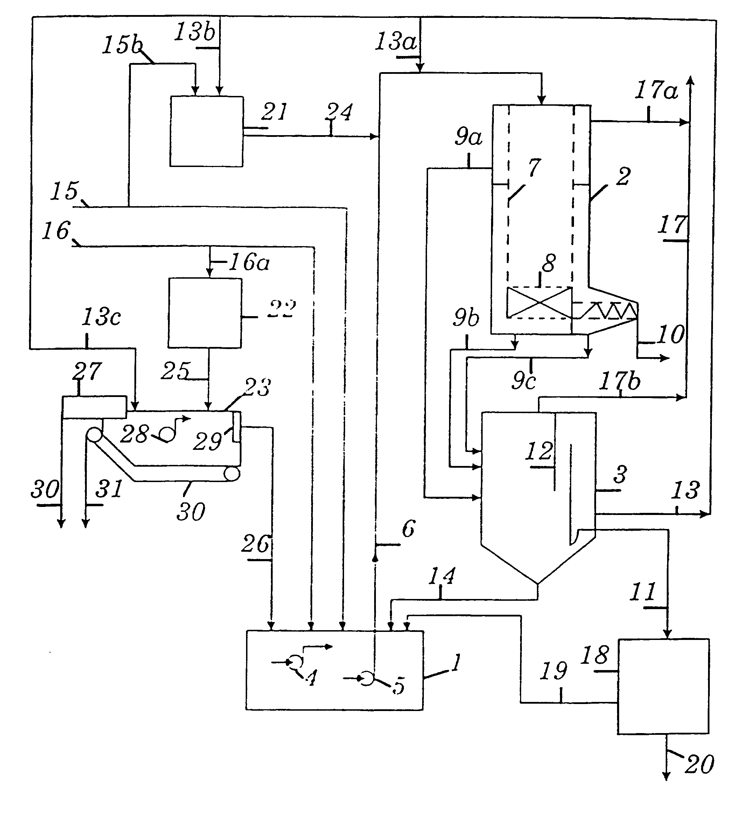

L'exemple d'installation conforme à l'invention représenté par la figure 2 correspond au traitement en mélange de matières biodigestibles de diverses origines, effluents pouvant requérir une correction de PH et/ou de concentration, solides, substrats concentrés et constituants non digestibles, boues de station d'épuration d'eau usée.

En amont de l'installation de méthanisation constituée de la cuve (1), de l'enceinte à solides et substrats concentrés (2) et de l'enceinte à liquides (3) on trouvera ,
un ensemble de préparation biochimique (21) d'effluents acheminés par la ligne (15b) par mélange avec des liquides issus de (3) acheminés par la ligne (13b) ; ce mélange est ensuite pompé par les lignes (24) et (6) vers l'enceinte (2),
un ensemble de préparation mécanique (22) d'un mélange de solides et substrats concentrés acheminés par la ligne (16a).

Ces matières sont envoyées par la ligne (25) à l'ensemble de préparation hydraulique (23) constitué d'un bain agité par l'agitateur (28) qui reçoit des liquides issus de l'enceinte (3) par la ligne (13c) ; le mélange de liquides et matières en solution et suspension s'écoule par le déversoir (29) et la canalisation (26) dans la cuve (1).

Les légers sont collectés par le dispositif (27) pour être évacués par le transporteur (32) ; les denses sont collectés par le dispositif (3) au fond de l'ensemble (23) pour être évacués par le transporteur (31).

Enfin la station d'épuration d'eaux usées (18) est associée à l'installation de méthanisation dont elle reçoit les jus résiduels isssus de l'enceinte (3) par la ligne (11); les boues d'épuration brutes produites par la station (18) sont envoyées directement à la cuve (1) par la ligne (19) ; les eaux épurées sortant de (18) sont évacuées par la ligne (20).

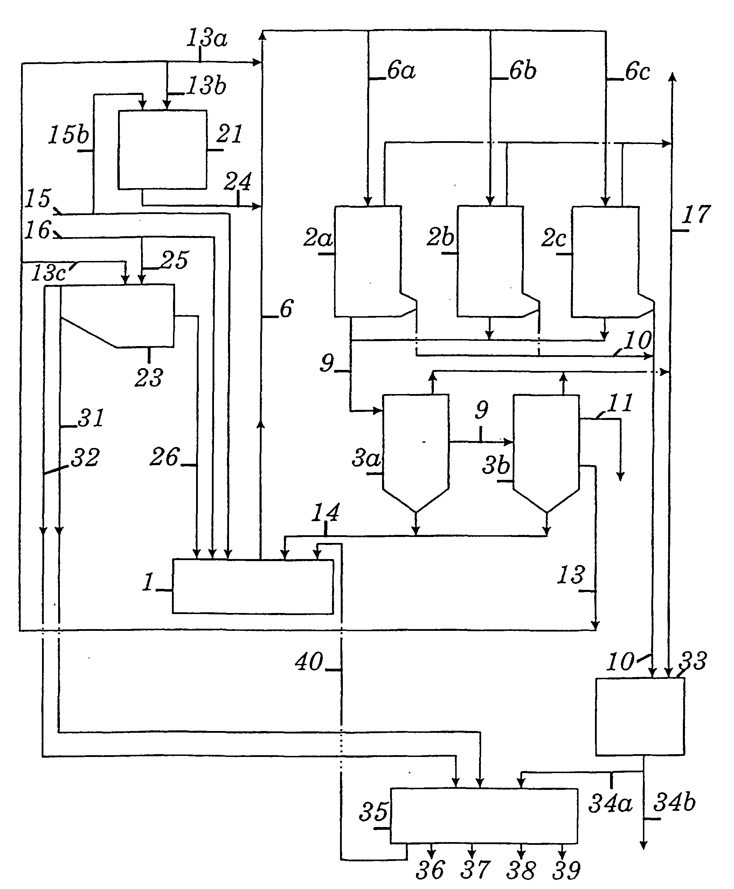

L'exemple d'installation conforme à l'invention représentée par la figure 3 comporte une installation de méthanisation ayant 3 enceintes à solides et substrats concentrés (2a), (2b) et (2c) et 2 enceintes à liquides (3a) et (3b).

Dans cet exemple, le résidu très peu aqueux issu des enceintes (2a), (2b), (2c), est acheminée par le transporteur (10) à l'étuve de séchage (33), alimentée en biogaz par le collecteur (17). Le résidu séché est, en tout ou partie, acheminé par le convoyeur (34a) à l'ensemble d'affinage (35), qui reçoit également les légers et les denses issus de la préparation hydraulique (23) par les transporteurs (32) et (31).

L'ensemble d'affinage (35) peut isoler :
- un compost anaérobie séché - affiné, évacué par le transporteur (36)
- des métaux réutilisables évacués par le transporteur (37)
- des plastiques réutilisables évacués par le transporteur (38)
- des déchets à éliminer évacués par le transporteur (39)
- les solides de grande taille contenus par le résidu séché pour les recycler grâce au transporteur (40) à la cuve (1) comme addition solide de «procédé » à la charge admise à la méthanisation.

## Revendications

1. Procédé de traitement des mélanges biodigestibles de solides et liquides comportant une installation de méthanisation dans laquelle la charge entrante est un mélange entre des matières à traiter de toute nature, à la composition et à la structure physique contrôlées et des liquides prélevés directement dans l'installation, la charge pouvant être complétée selon l'humidité et la structure physique des matières à traiter par des additions de liquide et de solides dites de procédé, la charge étant préparée dans une cuve (1) d'homogénéïsation-alimentation équipée d'au moins une pompe (5) hacheuse à liquide chargé de solides dans laquelle sont obtenus le mélange et un effet de hachage de la charge, la charge parvenant en partie haute d'une enceinte (2) dite à solides et substrats concentrés par pompage depuis la cuve (1) par la tuyauterie (6), l'enceinte (2) étant munie sur les flancs et le fond d'un cloisonnement perforé formant système (7) de drainage sélectif des liquides et en partie basse « d'un système (8) de récolte des solides et substrats concentrés résiduels qui essore et extrait de l'enceinte (2) un résidu très peu aqueux, le transit de la charge dans l'enceinte (2) étant gravitaire et contrôlé par son admission et l'extraction du résidu très peu aqueux, qui peuvent être continues ou discontinues, de façon à ce que se constituent dans l'enceinte (2) deux zones superposées la zone supérieure où il y a séparation rapide solides/liquides par flottation décantation et filtration par le lit de solides en place, la zone inférieure où la séparation solides/liquides est obtenue par filtration par les solides en place et libération de liquides circulants par digestion de matières digestibles, les liquides drainés par le dispositif (7) étant acheminés sélectivement par le dispositif de collecte (9) à l'enceinte (3) dite à liquide, l'enceinte (3) comportant un cloisonnement (12) pour obtenir des mouvements de liquide à l'intérieur de l'enceinte (3) induits par les entrées et sorties de liquides et par la gravité correspondant à un parcours méthodique des liquides assurant la séparation de liquides très peu chargés de matières en suspension qui sont évacuées hors de l'enceinte (3) par le dispositif de trop plein (11), d'avec les autres liquides qui sont recyclés à l'enceinte (2) par la ligne (14) vers la cuve (1) puis la ligne (6) ou directement par la ligne (13) puis la ligne (6), procédé **caractérisé par le fait que** l'installation de méthanisation comporte une cuve désignée par enceinte à solides et substrats concentrés qui reçoit une charge convenablement préparée à son sommet, munie d'un système sélectif de drainage de liquide sur les flancs et le fond et d'un système de récolte des solides et substrats concentrés en partie basse extrayant un résidu très peu aqueux, une cuve désignée par enceinte à liquides recevant par un dispositif sélectif de collecte, extérieur aux 2 cuves, les liquides drainés dans l'enceinte à solides et substrats concentrés, enceinte à liquide équipée d'un cloisonnement interne de façon à ce que par la conjugaison du système de drainage de l'enceinte à solides et substrats concentrés, du système de collecte de liquides reliant les 2 cuves, du cloisonnement interne à l'enceinte à liquides, des entrées et sorties de liquide dans et hors de cette enceinte, le parcours des liquides dans l'enceinte à liquides soit méthodique, assurant l'extraction par trop plein d'un liquide résiduel très peu chargé de matières en suspension hors de l'unité de méthanisation, les matières après méthanisation étant transformées en 3 sortants, du biogaz, des liquides très peu chargés en matières en suspension, des solides résiduels rassemblés en un résidu très peu aqueux.

2. Procédé de traitement des mélanges biodigestibles de solides et liquides selon la revendication 1, **caractérisé par le fait que** l'installation de méthanisation comporte plus d'une enceinte (2) à solides et substrats concentrés, ces enceintes ayant toutes les mêmes fonctions avec autant de points d'extraction de résidu très peu aqueux.

3. procédé de traitement des mélanges biodigestibles de solides et liquides selon l'une ou l'autre revendication 1 et 2 **caractérisé par le fait que** l'installation de méthanisation comporte plus d'une enceinte (3) à liquides, ces enceintes pouvant avoir 2 à 2 les mêmes fonctions ou des fonctions complémentaires, de façon à obtenir un parcours méthodique des liquides dans les enceintes et entre les enceintes, le rejet de liquides résiduels, s'opérant par un point unique de l'installation de méthanisation.

4. Procédé de traitement des mélanges biodigestibles de solides et liquides selon l'une ou l'autre des revendications 1, 2, 3 **caractérisé par le fait que** tout ou partie des matières traitées transitent par une unité de préparation hydraulique (23) par mélange à des liquides prélevés directement dans l'installation de méthanisation, pour séparer les matières brutes ou mécaniquement préparées en 3 parties, les matières de faible densité, qui flottent, les matières de haute densité, qui décantent, les matières en solution ou suspension qui seules envoyées à l'installation de méthanisation.

5. Procédé de traitement des mélanges biodigestibles de solides et liquides selon l'une ou l'autre des revendications 1, 2, et 3, **caractérisé par le fait que** tout ou partie des matières traitées sont des effluents ou boues légères dont le PH et/ou la concentration en éléments potentiellement inhibiteurs de la digestion sont corrigés par mélange avec des liquides prélevés directement dans l'installation de méthanisation, dans un ensemble de préparation biochimique (21), avant d'être envoyées à l'installation de méthanisation.

6. Procédé de traitement des mélanges biodigestibles de solides et liquides selon l'une ou l'autre des revendications 1, 2 et 3, **caractérisé par le fait que** l'installation de méthanisation est associée à une station d'épuration d'eaux usées (18), la station (18) recevant directement les liquides résiduels de la méthanisation pour les traiter seuls, ou en mélange avec d'autres effluents, les boues d'épuration issues de la station (18) étant envoyées directement à la cuve (1) pour être traitées par méthanisation seules, avec additions de solides de procédé, ou en mélange avec d'autres matières.

7. Procédé de traitement des mélanges biodigestibles de solides et liquides selon l'une ou l'autre des revendications 1, 2, 3, 4, 5, 6, **caractérisé par le fait que** le tri du résidu très peu aqueux issu de la méthanisation par l'ensemble d'affinage (35) permet de récupérer les solides résiduels de taille significative en vue de les recycler en tout ou partie dans la cuve (1) comme addition solide de procédé.

8. Procédé de traitement des mélanges biodigestibles de solides et liquides selon l'une ou l'autre des revendications 1, 2, 3, 5, 6 et 7, **caractérisé par le fait que** les matières traitées ne contiennent pas de solides de taille significative, et que l'addition de solides « de procédé » est faite avec des solides recyclés, non digestibles ou partiellement digestibles, sauf compensation des pertes avec des solides de même nature.

## Patentansprüche

1. Behandlungsverfahren von biologisch abbaubaren Stoff/Flüssigkeit Mischungen in einer Biogasanlage, in dem die zu behandelnde Masse aus einer Mischung von Stoffen verschiedenster Natur, deren physikalische Strukturen und Zusammensetzung analysiert sind, und direkt aus der Anlage entnommenen Flüssigkeiten besteht; je nach Feuchtigkeit und physikalischer Struktur der zu behandelnden Masse kann die Masse mit Flüssigkeit oder Feststoffen aus dem Prozess angereichert werden;
in einem mit Feststoffen gefüllten und mit mindestens einer Schneidpumpe (5) ausgestatteten Misch- und Beschickungsschacht (1) wird die Masse zerkleinert und vermischt; über die Rohrleitung (6) wird die Masse aus dem Misch- und Beschickungsschacht (1) in den oberen Teil des Behälters (2) des so genanten Behälters für Feststoffe und konzentrierte Substrate gepumpt;
der Behälter (2) ist innerhalb und am Boden mit perforierten Trennwänden versehen, die das selektive Entwässerungssystem gestalten (7); im unteren Teil des Behälters (2) befindet sich ein Sammelsystem für Feststoffe und konzentrierte Substrate mit Abzug für den getrockneten, wenig wasserhaltigen Endfeststoff;
der Stoffdurchlauf in dem Behälter (2) erfolgt durch die Schwerkraft der Last, die durch Stoffeinspeisung und Abfluss des wenig wasserhaltigen Endfeststoffs, die kontinuierlich oder diskontinuierlich sein können, reguliert wird;
der Behälter (2) ist in zwei übereinander stehende Bereiche aufgeteilt; im oberen Bereich erfolgt durch Flotation, Dekantieren und Filtrieren eine schnelle Trennung von Feststoffen und Flüssigkeiten über die vorhandene Feststoffschicht; im unteren Bereich findet durch Filtrieren über die vorhandene Feststoffschicht und Freisetzung der umlaufenden Flüssigkeiten aus der Vergärung abbaubarer Stoffe eine weitere Trennung von Feststoffen und Flüssigkeiten statt;
die über die Vorrichtung (7) freigesetzten Flüssigkeiten werden selektiv durch die Sammelvorrichtung (9) in den Flüssigkeitsbehälter (3) geleitet; der Behälter (3) ist mit inneren Trennwänden (12) ausgestattet, die einem methodischen Verlauf der Flüssigkeitsbewegungen im Behälter (3) dienen, der durch den Ein- und Ausfluss der Flüssigkeiten und durch die Schwerkraft bestimmt wird; er stellt die Trennung der Endflüssigkeiten mit sehr geringem Gehalt an Schwebstoffen sicher, die über ein Überlaufrohr aus dem Behälter (3), getrennt von den anderen Flüssigkeiten, die über die Verbindung (14) in Richtung Schacht (1) weiter über Verbindung (6 ) oder direkt über die Verbindung (13) und weiter über (6) in dem Behälter (2) wieder verwendet werden, abgeleitet werden;
das Verfahren ist **dadurch gekennzeichnet**, das die Biogasanlage über einen Behälter für Feststoffe und konzentrierte Substrate verfügt, der eine angemessen vorbereitete Masse von oben erhält; er ist innerhalb und am Boden mit einem selektiven Entwässerungssystem und einer Sammelvorrichtung für Feststoffe und konzentrierte Substrate im unteren Bereich ausgestattet, die einen wenig wasserhaltigen Endfeststoff auswirft;
ein Flüssigkeitsbehälter erhält über eine selektive Sammelvorrichtung, ausserhalb der 2 Behälter, die Flüssigkeiten aus dem Behälter für Feststoffe und konzentrierte Substrate ; der Flüssigkeitsbehälter ist innen mit Trennwänden in der Art ausgestattet, das durch die Verbindung des Entwässerungssystem im Behälter für Feststoffe und konzentrierte Substrate, mit dem Flüssigkeitssammelsystem, das die 2 Behälter verbindet, mit den inneren Trennwänden im Flüssigkeitsbehälter und mit den Flüssigkeitsein- und Abflüssen in oder ausserhalb dieses Behälters, der methodische Durchlauf der Flüssigkeiten im Flüssigkeitsbehälter und über ein Überlaufrohr der Abzug, ausserhalb der Biogasanlage, einer Endflüssigkeit mit einem geringen Gehalt an Schwebstoffen garantiert wird;
nach der Vergärung verbleiben 3 Endprodukte, das Biogas, eine geringfügig mit Schwebstoffen belastete Endflüssigkeit, ein fester, wenig wasserhaltiger Endfeststoff.

2. Behandlungsverfahren biologisch abbaubarer Stoff/Flüssigkeit Mischungen nach dem Anspruch 1, **gekennzeichnet dadurch**, das die Ausstattung der Biogasanlage mehrere Behälter für Feststoffe und konzentrierte Substrate (2) umfasst; diese Behälter haben die gleichen Funktionen und sind jeweils mit einem Abzug für den wenig wasserhaltigen Endfeststoff versehen;

3. Behandlungsverfahren biologisch abbaubarer Stoff/Flüssigkeit Mischungen nach Anspruch 1 oder 2 **gekennzeichnet dadurch**, das die Biogasanlage mehrere Flüssigkeitsbehälter (3) umfasst; die Behälter können paarweise die gleichen oder ergänzende Funktionen haben, um einen methodischen Durchlauf der Flüssigkeiten in den Behältern und zwischen den Behältern zu gewährleisten; der Endflüssigkeitsabzug erfolgt an einer einzigen Stelle der Biogasanlage;

4. Behandlungsverfahren biologisch abbaubarer Stoff/Flüssigkeit Mischungen nach Anspruch 1, 2, oder 3, **gekennzeichnet dadurch, dass** alle Stoffe oder Teile davon durch eine hydraulische Vorbereitungseinrichtung laufen und mit direkt aus der Biogasanlage entnommenen Flüssigkeiten vermischt werden, um so die unbehandelten oder mechanisch vorbereiteten Stoffe in 3 Komponenten zu trennen, nämlich Schwimmstoffe mit geringer Dichte, Stoffe mit hoher Dichte, die dekantiert werden, und gelöste- oder Schwebstoffe, die allein der Biogasanlage zugeführt werden.

5. Behandlungsverfahren biologisch abbaubarer Stoff/Flüssigkeit Mischungen nach Anspruch 1, 2, und 3, **gekennzeichnet dadurch**, das der pH-Wert und oder die potentielle Vergärungshemmstoffkonzentration aller bzw. teilweise behandelter Stoffe, die aus Stoffen oder Schlamm geringfügiger Dichte bestehen, durch Vermischung mit direkt aus der Biogasanlage entnommenen Flüssigkeiten in einer biochemischen Vorbereitungseinheit vor Einspeisung in die Biogasanlage korrigiert werden;

6. Behandlungsverfahren biologisch abbaubarer Stoff/Flüssigkeit Mischungen nach Anspruch 1, 2, 3, **gekennzeichnet dadurch, dass** die Biogasanlage mit einer Kläranlage verbunden ist, die direkt von der Biogasanlage die Endflüssigkeiten erhält, um es allein oder in Vermischung mit anderem Abwasser zu behandeln; der anfallende Klärschlamm wird direkt von der Kläranlage in den Misch- und Beschickungsschacht (1) geleitet, wo er allein oder in Vermischung mit Prozessfeststoffen oder in Vermischung mit anderen Stoffen zur Vergärung vorbereitet wird.

7. Behandlungsverfahren biologisch abbaubarer Stoff/Flüssigkeit Mischungen nach Anspruch 1, 2, 3, 4, 5 oder 6, **gekennzeichnet dadurch**, das in der Aufbereitungseinheit (35) grosse Feststoffteile vom wenig wasserhaltigen Vergärungsendfeststoff getrennt werden und ganz oder teilweise als zusätzlicher fester Prozessstoff zum Misch- und Beschickungsschacht (1) weitergeleitet werden können.

8. Behandlungsverfahren biologisch abbaubarer Stoff/Flüssigkeit Mischungen nach Anspruch 1, 2, 3, 5, 6 oder 7, **gekennzeichnet dadurch, dass** die zu behandelnden Stoffe keine grossen Feststoffteile enthalten und dass der Feststoffzusatz aus nicht oder teilweise abbaubaren Prozessfeststoffen besteht, ausser dem aus der Verlustkompensierung gleichartiger Materialien

## Claims

1. Method of treating biodigestible mixtures of solids and liquids comprising a Methanization Plant in which the incoming load to be treated is a mixture of matter of all kinds, of which the composition and physical structure are checked, with liquid drawn directly from the Plant itself, said load possibly adjusted, depending on its moisture content and physical structure, using so-called process addition solids and liquids, which load is prepared in homogenization-feed Tank (1) fitted with at least one chopping Pump (5) handling liquids containing suspended particles, in which Tank mixing and chopping effects are obtained, the load being pumped via line (6) from Tank (1) into the top of solids and concentrated feedstock Vessel (2), the sidewalls and bottom of said Vessel (2) being fitted with a perforated partition making up selective draining System (7) and the lower part being fitted with a solids and concentrated feedstock gathering System (8) which dries a load passing through Vessel (2) by gravity, controlled by inputting and extraction of low water-content residue, either continuously or by batches, and extracts low water-content residue under pressure from Vessel (2) such that two superimposed zones are created in Vessel (2), an upper zone for quick liquid - solid separation by floating, sinking and filtration in the solid bed, a lower zone for liquid - solid separation by filtration by the solid bed and release of free liquids by digestion of digestible matter, the liquids drained by System (7) being carried selectively by collecting System (9) to liquids Vessel (3), said Vessel (3) fitted with partition (12) to subject the movement of liquids inside Vessel (3) to the effect of liquid inputs and outputs and gravity, in order to achieve a methodical run ensuring separation of liquids containing little suspended matter, said liquids being discharged from Vessel (3) via overflow (11), from other liquids recycled by Vessel (2) via line (14) to Tank (1) and then line (6) or directly via line (13) and then line (6), Method **characterized by** the fact that the Methanization Plant has a {Vessel designated as } solids and concentrated feedstock Vessel which takes in a suitably prepared load by the top, is fitted on the sidewalls and bottom with a selective drainage system and on the lower part with a solids and concentrated feedstock gathering system which dries and extracts low water-content residue under pressure, a {Vessel designated as} liquids Vessel taking in liquids drained from the solids and concentrated feedstock Vessel via one selective collecting system external to both Vessels, the liquids Vessel being fitted with an internal partition to achieve a methodical liquids run in the liquids Vessel through a combination of the draining system equipping the solids and concentrated feedstock Vessel, the liquid collecting system connecting the two Vessels, the partition inside the liquids Vessel, the liquid inputs into and outputs from said Vessel, to ensure discharge of residual liquid containing the least possible suspended particles from the Methanization Plant, processed matter being converted into 3 outputs, biogas, liquids containing very few suspended particles, residual solids composing low water-content residue.

2. Method of treating biodigestible mixtures of solids and liquids according to claim 1, **characterized by** the fact that the Methanization Plant comprises more than one solids and concentrated feedstock Vessel (2), said Vessels performing identical duties and having as many low water-content residue discharge point as Vessels.

3. Method of treating biodigestible mixtures of solids and liquids according to either claim 1 or claim 2, **characterized by** the fact that the Methanization Plant comprises more than one liquids Vessel (3); said Vessels may be paired to perform the same duty or complementary duties, such that the liquids run motion inside each Vessel and between Vessels is methodical, residual liquids being discharged from the Methanisation Plant via a single point.

4. method of treating biodigestible mixtures of solids and liquids according to either claim 1, claim 2 or claim 3, **characterized by** the fact that all or part of the processed matter passes through hydraulic preparation Unit (23) to be mixed with liquids drawn directly from the Methanization Plant to separate raw or mechanically prepared matter into three fractions; floating low-density matter, sinking high-density matter, dissolved or suspended matter which alone is transferred to the Methanization Plant.

5. Method of treating biodigestible mixtures of solids and liquids according to either claim 1, claim 2 or claim 3, **characterized by** the fact that all or part of the processed matter are effluents or slurries, subject to adjustment of pH and/or concentration of potential digestion inhibiting elements by mixing with liquids drawn directly from the Methanization Plant in biochemical preparation Unit (21) before transfer to the Methanization Plant.

6. Method of treating biodigestible mixtures of solids and liquids according to either claim 1, claim 2 or claim 3, **characterized by** the fact that the Methanization Plant is associated with wastewater treatment Plant (18), said plant (18) receiving residual liquids directly from the Methanization Plant for processing, either alone or mixed with other effluents, the sludge from plant (18) being carried directly to Tank (1) for processing by methanization alone, by addition of process solids or by mixture with other matter.

7. Method of treating biodigestible mixtures of solids and liquids according to either claim 1, claim 2, claim 3, claim 4, claim 5 or claim 6, **characterized by** the fact that sorting of low water-content residue from methanization in refining Unit (35) enables residual solids of significant size to be recovered for full or partial recycling in Tank (1) as solid process additions.

8. Method of treating biodigestible mixtures of solids and liquids according to claim 1, claim 2, claim 3, claim 5, claim 6 or claim 7, **characterized by** the fact that processed matter does not contain solids of significant size and that non-digestible or partially digestible recycled solids, are used for process solid addition, except for solids of the same kind to compensate for losses.
